# NOUVEAU FASCICULE DE BREVET EUROPEEN

(11) **EP 0 487 160 B2**
(45) Date de publication et mention de la décision concernant l'opposition: **07.11.2001**
(45) Mention de la délivrance du brevet: 23.03.1994
(21) Numéro de dépôt: 91203010.3
(22) Date de dépôt: 19.11.1991
(51) Int. Cl.: C07C 249/16, C07C 251/08, C01B 21/16

(54) **Procédé de synthèse d'azines**
Verfahren zur Herstellung von Azine
Process for the preparation of azines

(30) Priorité: 23.11.1990 FR 9014634
(43) Date de publication de la demande: 27.05.1992
(73) Titulaire: Atofina, 92800 Puteaux (FR)
(72) Inventeur: Schirmann, Jean-Pierre, F-69600 Oullins (FR); Tellier, Pierre, F-69110 Sainte Foy les Lyon (FR)

(56) Documents cités:
- DE-A- 2 639 009
- US-A- 3 869 541
- US-A- 3 943 152
- US-A- 3 972 876
- US-A- 4 093 656
- Ullmann's Encyclopedia of Industrial Chemistry, 5th Ed. vol. A13, 1989, pp. 182-183
- Organic Synthesis, J. Wiley and Sons, Inc., New York, Coll. Vol. 1, p. 3-4
- Oranic Synthesis, J. Willey and Sons, Inc., New York, Coll. Vol. 1, p. 3-4

## Description

La présente invention concerne un procédé de synthèse d'azines et son application à la production d'hydrazine. L'hydrazine, le plus souvent sous la forme d'hydrate d'hydrazine est un produit très utilisé en tant que tel ou comme intermédiaire de synthèse. Selon ULLMANN - 5e édition 1989 - Vol A 13 pages 177-191, l'hydrazine est produite soit par oxydation de l'ammoniac à l'aide de chlore ou d'eau de javel éventuellement en passant par une cetazine, soit par réaction de l'eau oxygénée d'ammoniac et d'une cétone.

La présente invention concerne le procédé à l'eau oxygénée ; il a été décrit dans de nombreux brevets, notamment US 3 972 878, US 3 972 876, US 3 869 541, US 3 948 902, US 3 919 256, US 3 943 152, US 4 093 656.

Selon ce procédé on fait la réaction de l'ammoniac, de l'eau oxygénée et d'un produit portant un groupe 〉C = O tel qu'une cétone qu'on peut représenter par l'équation suivante :

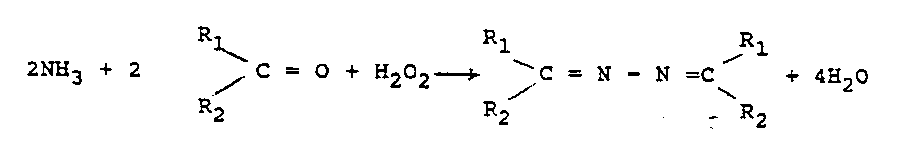

La réaction s'effectue en présence de catalyseur.

L'azine peut être ensuite hydrolysée en hydrate d'hydrazine selon l'équation : 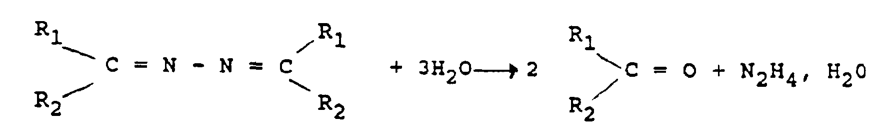 en régénérant la cétone.

Dans le brevet US 3 972 878 il est décrit une réaction en présence d'un mélange d'un amide d'un acide faible et d'un phosphate. On a utilisé par exemple de l'acétamide avec du phosphate de lithium, de l'acétamide avec du carbonate de sodium, du propionamide avec du phosphate de lithium, de l'acétamide avec du phosphate disodique, de l'acétamide avec du phosphate de diisopropyl.

Le rendement en azine basé sur l'eau oxygénée est entre 45 et 55 % et monte à 78 % pour le couple acétamide, phosphate disodique.

Dans le brevet US 4 093 656 il est décrit aussi dans une réaction de synthèse d'azines un catalyseur constitué d'un mélange de trois produits à savoir un amide d'un acide faible, le sel d'ammonium correspondant à cet acide et un autre produit tel qu'un phosphate. On a utilisé par exemple de l'acétamide, de l'acétate d'ammonium et du phosphate de sodium. Le rendement en azine basé sur l'eau oxygénée atteint 85 %. On a maintenant trouvé une importante simplification à savoir qu'on observe le même rendement quand on utilise soit un catalyseur constitué d'un mélange d'un amide d'acide faible, du sel d'ammonium correspondant et d'un produit tel qu'un phosphate soit un catalyseur constitué d'un mélange d'un amide d'acide faible et du sel d'ammonium correspondant.

La présente invention concerne donc un procédé de synthèse d'azines à partir d'eau oxygénée, d'ammoniac et d'un réactif portant un groupe carbonyle caractérisé en ce qu'on met en contact ces réactifs avec un amide d'acide faible et le sel d'ammonium correspondant à cet acide en l'absence de tout catalyseur mineral ou organique puis qu'on sépare l'azine obtenue.

L'eau oxygénée peut être utilisée sous la forme commerciale habituelle, par exemple en solution aqueuse entre 30 et 90 % en poids de H₂O₂. Avantageusement, on peut ajouter un ou plusieurs stabilisants usuels des solutions péroxydiques, par exemple le sel de sodium de l'acide éthylènediaminetétracétique. L'ammoniac peut être anhydre ou en solution aqueuse.

Ce réactif portant un groupe carbonyle est de formule 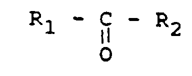 dans laquelle R₁ et R₂, identiques ou différents, représentent l'hydrogène, un radical alkyl ayant de 1 à 12 atomes de carbone, un radical alkyle ramifié ou cycloalkyl atomes de carbone, un radical alkyle ramifié ou cycloalkyl ayant de 3 à 12 atomes de carbone, un radical aromatique ayant de 6 à 12 atomes de carbone ou représentent ensemble un radical alkylène linéaire ou ramifié ayant de 3 à 12 atomes de carbone ; ces radicaux pouvant être substitués par un halogène, un groupe NO₂, hydroxy, alkoxy ou ester carboxylique, et de préférence Cl, NO₂ ou CH₃O.

Des exemples de réactifs : 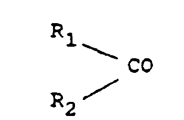 sont des aldéhydes ou des cétones.

Parmi les aldéhydes on peut citer le formaldéhyde, l'acétaldéhyde, le butyraldéhyde, l'isobutyraldéhyde, le n-pentanal, le pivalaldéhyde, le benzaldéhyde, les monochlorobenzaldéhydes, le paranitrobenzaldéhyde, l'anisaldéhyde, le betachloropropionaldéhyde, le beta-méthoxypropionaldéhyde.

Parmi les cétones, on peut citer l'acétone, la 2-pentanone, la 3-pentanone, la méthylisopropyl cétone, la méthylisobutyl cétone, la méthyléthyl cétone, la méthyl cyclohexyl cétone, l'acétophénone, la benzophénone, la cyclobutanone, la cyclopentanone, la cyclohexanone.

On utilise avantageusement les cétones dans lequelles R₁ et R₂ sont identiques ou différents, et sont des alkyls linéaires ou ramifiés ayant de 1 à 5 atomes de carbone et de préférence l'acétone, la méthyléthyl cétone et la méthyl isobutyl cétone.

Les réactifs peuvent être utilisés en quantités stoéchiométriques, cependant on utilise par mole d'eau oxygénée 0,2 à 5 moles et de préférence 1,5 à 4 moles du réactif carbonylé (aldéhyde ou cétone) et 0,1 à 10 moles et de référence 1,5 à 4 moles d'ammoniac.

Les amides de la présente invention sont dérivés des acides carboxyliques correspondants qui ont une constante de dissociation inférieure à 5 x 10⁻⁵, c'est-à-dire les acides qui ont un PK plus grand que 4,3 en solution aqueuse à 25°C.

Pour les acides polycarboxyliques ce sont les acides dont la constante de la première ionisation est inférieure à 5 x 10⁻⁵.

A titre d'exemple on peut citer les acides carboxyliques de formule R₅COOH dans laquelle R₅ est un radical alkyl linéaire ayant de 1 à 20 atomes de carbone, ou un radical alkyl ramifié ou cyclique ayant de 3 à 12 atomes de carbone, ou un radical phényl pouvant être substitué, des acides polycarboxyliques de formule R₆(COOH)ₙ dans laquelle R₆ représente un radical alkylène ayant de 1 à 10 atomes de carbone et n valant 1 ou 2, R₆ peut être une simple liaison alors n vaut 2. Les radicaux R₅ et R₆ peuvent être substitués par des halogènes, des groupements OH, NO₂ ou méthoxy. On utilise de préférence l'acétamide, le propionamide, le n-butyramide ou l'isobutyramide.

Le sel d'ammonium correspondant de l'acétamide est l'acétate d'ammonium.

On ne sortirait pas du cadre de l'invention en formant le sel d'ammonium in situ c'est à dire en utilisant l'acide carboxylique correspondant qui donne par réaction avec l'ammoniac le sel d'ammonium.

Les proportions de l'amide et du sel d'ammonium correspondant peuvent varier dans de larges limites. On utilise habituellement de 1 à 25 parties du sel d'ammonium pour 5 parties d'amide et de préférence 2 à 10.

La quantité de l'amide peut varier dans de larges limites.

La mise en contact de l'eau oxygénée, de l'ammoniac, du réactif portant un groupe carbonyle avec l'amide et le sel d'ammonium peut s'effectuer de façon quelconque.

Avantageusement on opère dans un milieu homogène ou dans un milieu qui assure au moins une solubilisation suffisante des réactifs pour pouvoir obtenir de l'azine. La réaction peut se faire dans une très large plage de température, par exemple entre O° et 100°C, et on opère avantageusement entre 30 et 70°C. Bien qu'on puisse opérer à toute pression, il est plus simple d'être à la pression atmosphérique, mais on peut monter jusqu'à environ 10 bar si c'est nécessaire pour maintenir de préférence la réaction en phase liquide.

Les réactifs peuvent être introduits simultanément ou séparément et dans un ordre quelconque. On peut utiliser toutes sortes de réacteurs, agités ou non agités, ou même de simples capacités qu'on peut disposer en parallèle en série à co-courant ou à contre-courant, ou toute autre combinaison de ces possibilités.

L'amide et le sel d'ammonium sont généralement utilisés sous forme d'une solution aqueuse.

Cette solution peut aussi comprendre un alcool. Parmi les alcools on utilise avantageusement les alcools aliphatiques saturés avant de 1 à 6 atomes de carbone et de référence 1 à 2 atomes de carbone.

On utilise aussi avantageusement des diols et plus particulièrement des diols ayant de 2 à 5 atomes de carbone. On peut citer par exemple le glycol, le propylèneglycol, le 1,3 propanediol, le 1,3 et 1,4 butanediol et le 1,5 pentanediol.

En fin de réaction on peut récupérer l'azine par distillation, extraction liquide-liquide ou tout moyen équivalent, ou même tout ou partie par simple décantation si elle est insoluble dans le milieu de réaction.

Après avoir récupéré l'azine du mélange réactionnel, il reste une solution contenant de l'amide et du sel d'ammonium et éventuellement de l'ammoniac, du réactif à groupe carbonyle, de l'eau oxygénée et divers sous-produits ou impuretés.

On récupère autant que possible l'ammoniac et le réactif à groupe carbonyl pour les recycler avantageusement à la réaction de synthèse.

L'avantage de l'invention est d'éviter l'emploi de produits tels que les phosphates qui compliquent le traitement ultérieur de la solution d'amide et de sel d'ammonium. L'eau oxygénée est difficile à recycler, on opère dans des conditions telles qu'on la consomme entièrement.

Que l'on effectue entièrement la synthèse d'azines (i) en présence d'un amide d'acide faible et du sel d'ammonium correspondant comme on vient de le décrire ou (ii) en présence d'un amide d'acide faible, du sel d'ammonium correspondant et d'un produit tel qu'un phosphate comme décrit dans le brevet US 4 093 656 ou (iii) en présence d'un amide d'acide faible et d'un produit tel qu'un phosphate comme décrit dans le brevet US 3. 972 878, la présente invention concerne aussi le traitement des solutions contenant ces produits pour leur recyclage dans une synthèse d'azines à partir d'eau oxygénée, d'ammoniac et d'un réactif portant un groupe carbonyle.

La présente invention concerne donc aussi un procédé de synthèse d'azines à partir d'eau oxygénée, d'ammoniac et d'un réactif portant un groupe carbonyle caractérisé en ce que
a) on met en contact ces réactifs avec un mélange contenant un amide d'acide faible
b) on sépare l'azine obtenue
c) on traite le mélange par chauffage entre 170 et 230°C pour régénérer la quantité initiale de l'amide du début de l'étage a
d) on recycle le mélange obtenu en fin de c à l'étape a.

Le réactif portant le groupe carbonyle est le même que celui décrit plus haut. Le mélange contenant l'amide est l'un des trois mélanges décrits plus haut c'est-à-aire qu'il contient aussi un produit pouvant être un phosphate comme décrit dans US 3 972 878 ou contenant en plus le sel d'ammonium correspondant comme décrit dans US 4 093 656 ou bien c'est le mélange d'amide d'acide faible et de sel d'ammonium correspondant comme décrit au début de ce texte.

La demanderesse vient de découvrir que à l'issue de l'étape a une partie de l'amide s'était transformée en sel d'ammonium correspondant, c'est-à-dire que si on engage de l'acétamide dans l'étape a on obtient de l'acétamide et de l'acétate d'ammonium.

Comme précédemment après l'étape a de synthèse on récupère l'azine en b et il reste une solution contenant une partie de l'amide. engagé et du sel d'ammonium correspondant à cet amide. Le nombre de moles de l'amide restant ajouté au nombre de moles de sel d'ammonium correspondant est sensiblement égal au nombre de moles d'amide engagé en a. Cette solution contient aussi en plus le produit qui accompagnait l'amide au début de l'étape a, ce produit peut être le sel d'ammonium correspondant ou du phosphate ou un mélange des deux (sel et phosphate) c'est-à-dire que si on avait en a au début de réaction de l'acétamide et du phosphate on obtient une solution d'acétamide, d'acétate d'ammonium et de phosphate. Si on avait en a au début de l'acétamide et de l'acétate on obtient une solution avec moins d'acétamide et plus d'acétate. Si on avait en a au début de l'acétamide de l'acétate et du phosphate on obtient une solution avec moins d'acétamide, plus d'acétate et du phosphate. Cette solution peut aussi contenir, après qu'on ait enlevé l'azine, des impuretés et des réactifs en excédent : ammoniac, réactif portant un groupe carbonyle. Il est avantageux de récupérer ces réactifs en excédent. On effectue ensuite l'étape c, c'est-à-dire qu'on transforme le sel d'ammonium en amide correspondant jusqu'à régénérer l'amide initial de a. Un moyen avantageux consiste par exemple en un simple chauffage. Cette réaction est connue en soi. En effectuant cette étape c par chauffage, on obtient donc le retour du sel d'ammonium en amide pour reconstituer le mélange contenant un amide de l'étape a. Cette réaction peut aussi s'accompagner d'une décomposition d'une partie du sel d'ammonium en ammoniac et en acide correspondant.

On remélange l'ammoniac et l'acide pour reformer du sel d'ammonium qu'on recycle.

Si le mélange à reconstituer pour effectuer l'étape a est un mélange contenant un amide et le sel d'ammonium correspondant alors il est pas gênant que la régénération de l'étape c produise un mélange d'amide et d'acide correspondant. En effet il suffit d'ajouter de l'ammoniac à ce mélange avant de le recycler à l'étape a ou mieux de prévoir un excédent d'ammoniac dans l'étape a pour reformer ce sel d'ammonium in-situ. On peut se reporter au JACS (J of American Chemical Society) Mai 1931, pages 1879 à 1883. On chauffe à au moins 160° et de préférence entre 170 et 230°. De préférence on effectue le chauffage en distillant de l'eau. Si le mélange à reconstituer pour effectuer l'étape a est un mélange contenant un amide et le sel d'ammonium correspondant, l'opération de l'étage c consiste donc à transformer le mélange amide/sel d'ammonium qui s'est appauvri en amide et enrichi en sel d'ammonium au cours de a par conversion d'une partie du sel en amide. On ne sortirait pas du cadre de l'invention si on effectue l'étape c seulement sur une partie de la solution c'est à dire qu'après l'étape b une partie seulement de la solution riche en sel d'ammonium et pauvre en amide est traitée en c puis est remélangée avec la partie non traitée. Comme précédemment une partie du sel d'ammonium peut être sous forme d'acide on ajoute alors de l'ammoniac pour retrouver le sel s'ammonium.

On effectue alors l'étape d. On constate qu'en recyclant cette solution à l'étape a on peut produire de l'azine. Si on n'effectue pas ce traitement de l'étape c avant d'effectuer d alors on obtient une production d'azines plus faible qu'au cours de la synthèse précédente et qu'après 2 ou 3 recyclages la production est très faible.

Selon une forme préférée de l'invention, on peut éliminer certaines impuretés à haut point d'ébullition de la solution obtenue à la fin de c. L'élimination de ces impuretés à haut point d'ébullition peut se faire tout simplement par distillation. Cette quantité d'impuretés représente entre 0,01 et 5 % en poids de la totalité de la solution.

Cette quantité est faible et peut dépendre des conditions de la synthèse d'azines, des impuretés déjà présentes dans l'ammoniac, l'eau oxygénée et le réactif portant un groupe carbonyle et on sait que l'eau oxygénée dans ces conditions peut conduire à des impuretés lourdes.

Au lieu de procéder par distillation, on peut aussi éliminer ces impuretés par passage sur des matières absorbantes constituées de particules microporeuses. On peut utiliser par exemple les produits décrits dans le brevet US 4 657 751 avant de recycler la solution contenant l'amide et éventuellement le sel d'ammonium correspondant. On effectue si nécessaire l'appoint en phosphate ou en tout additif qui accompagnait l'acide et le sel d'ammonium lors de l'étape a.

Il est bien clair que l'étape c consiste à régénérer la partie de l'amide qui s'est transformée en sel d'ammonium correspondant. Si lors de l'étape a on a engage de l'amide et du sel d'ammonium correspondant on ne regénère qu'une fraction du sel d'ammonium de manière à retrouver le mélange initial d'amide et de sel d'ammonium.

### Exemple 1 :

Dans un réacteur de 250 ml muni d'un agitateur, d'un réfrigérant et d'un tuble plongeant relié à une réserve d'ammoniac on introduit les réactifs suivants :
- eau : 29 grammes
- acétamide : 59 grammes (1 mole)
- acétate d'ammonium : 64 grammes (0,83 mole)
- sel de sodium de l'acide éthylène diamine tétracétique : 0,1 gramme (stabilisant de H₂O₂)
- méthyléthyl cétone : 72 grammes (1 mole)

Par le tube plongeant on introduit de l'ammoniac gazeux jusqu'à saturation et on porte la température à 50°C.

En maintenant le courant d'ammoniac on introduit alors en 30 minutes 24,3 grammes d'une solution aqueuse à 70 % en poids de peroxyde d'hydrogène (0,5 mode).

Après 8 h 30 de réaction on refroidit le réacteur et on sépare les deux phases (organique et aqueuse) par décantation. On dose dans chacune d'elles le peroxyde d'hydrogène (par iodométrie) et l'azine (par chromatographie en phase vapeur).

La phase aqueuse contient 0,016 mode de peroxyde d'hydrogène et 0,018 mole d'azine de la méthyl éthyl cétone.

La phase organique contient 0,005 mole de peroxyde
Conversion du peroxyde d'hydrogène : 95,8 %
Sélectivité : 86 %
Rendement : 82,4 %

### Exemple 2 (comparatif):

On opère comme dans l'exemple 1 mais on ajoute en plus au départ 0,5 gramme de phosphate disodique.

Après 8 h 30 de réaction la phase aqueuse contient 0,023 mole de peroxyde d'hydrogène et 0,014 mole d'azine. La phase organique contient 0,003 mode de peroxyde d'hydrogène et 0.391 mole d'azine.
Conversion du peroxyde d'hydrogène : 96,8 %
Sélectivité : 83.7 %
Rendement : 81 %

### Exemple 3 :

On effectue une synthèse d'azine avec la méthyléthyl cétone et un mélange d'acétamide et de phosphate disodique, à la fin de l'étape a l'azine de la MEK est insoluble, on la sépare par décantation et il reste une phase aqueuse.

La phase aqueuse sortant des réacteurs est soumise à un lavage par de la méthyléthylcétone revenant de l'hydrolyse afin d'extraire l'azine qui est contenue. Une nouvelle phase aqueuse est obtenue. Elle correspond à la composition moyenne suivante (% massiques) :

| | |
|---|---|
| H₂O₂ = | 0,2 % |
| NH₃ = | 5,2 % |
| MEK* = | 3,5 % |
| Acétamide = | 19,7 % |
| Acétate d'ammonium = | 32,5 % |
| H₂O = | 38,8 % |
| Azine = | 0,1 % |
| Impuretés = | le complément |

| | |
|---|---|
| * *MEK = Méthyléthyl cétone* | |

On traite (étape c) la moitié de cette phase aqueuse.

### Appareillage utilisé

L'appareillage de laboratoire utilisé est constitué :
. d'une colonne Oldershaw adiabatique de laboratoire à plateaux perforés de diamètre intérieur O = 20 mm, et comportant 20 plateaux
. d'un bouilleur en titane de volume 250 cm3
. d'un condenseur
. d'un pot de reflux
. de pompes doseuses à piston permettant d'assurer l'introduction du mélange, le soutirage du bouilleur et l'introduction du reflux
. le reflux est déterminé grâce à un débitmètre à flotteur préétalonné
. le chauffage du bouilleur est assuré par un bain métallique maintenu à 200°C grâce à une régulation de température
. la canalisation de soutirage ainsi que la tête de la pompe de soutirage sont maintenues à 80° grâce à une double enveloppe dans laquelle circule de l'huile chaude, de façon à éviter toute prise en masse.

### Conditions opératoires

L'introduction du mélange à traiter est faite dans le bouilleur au débit de 349,5 g/heure.

En marche normale les températures sont les suivantes:

| | |
|---|---|
| Bouilleur | 180-181° |
| Plateau 10 | 135° |
| Tête | 94-95% |

Le taux de reflux est de 0,1.

Le temps de séjour dans le bouilleur est de 1 h 30.

On recueille en tête 190,5 g/heure d'un distillat de composition :

| | |
|---|---|
| NH₃ | 14,7 % |
| MEK | 6,6 % |
| H₂O | 78,5 % |
| Azine | 0,1 % |
| Acétate | 0 |

On obtient par soutirage du bouilleur 159 g/heure d'une solution de composition :

| | |
|---|---|
| Acide acétique | 21,5 % |
| Acétate | 2,6 % |
| Acétamide | 73,6 % |
| H₂O | 2,2 % |
| MEK | traces |
| Azine | traces |

Ce soutirage du bouilleur est remélangé à l'autre moitié non traitée puis renvoyée à l'étape a.

L'ammoniac recueilli en tête est partiellement séparé de l'eau puis est recyclé aussi en a.

## Revendications

1. Procédé de synthèse d'azines à partir d'eau oxygénée d'ammoniac et d'un réactif portant un groupe carbonyle **caractérisé en ce que** :
a) on met en contact ces réactifs avec un mélange contenant un amide d'acide faible
b) on sépare l'azine obtenue
c) on traite le mélange par chauffage entre 170 et 230°C pour régénérer la quantité initiale de l'amide du début de l'étape a
d) on recycle le mélange obtenu en fin de c à l'étape a.

2. Procédé selon la revendication 1 **caractérisé en ce que** le réactif portant un groupe carbonyle est une cétone et de préférence l'acétone, la méthyléthyl cétone ou la méthylisobutyl cétone.

3. Procédé selon la revendication 1 ou 2 **caractérisé en ce que** l'amide est choisi parmi l'acétamide, le proponiamide, le n-butyramide ou l'isobutyramide.

4. Procédé selon les revendications 1 à 3 **caractérisé en ce que** dans l'étape a on utilise en plus de l'amide un sel d'ammonium correspondant à l'acide dont est dérivé l'amide.

## Patentansprüche

1. Verfahren zur Synthese von Azinen aus Wasserstoffperoxid, Ammoniak und einem Reagenz mit einer Carbonylgruppe, **dadurch gekennzeichnet, daß**:
a) man diese Reagenzien mit einem Gemisch zusammengibt, das ein Amid einer schwachen Säure enthält
b) man das erhaltene Azin abtrennt
c) man das Gemisch durch Erhitzen zwischen 170 und 230 °C behandelt, um die Ausgangsmenge des Amids am Anfang von Schritt a zurückzugewinnen
d) man das am Ende von c erhaltene Gemisch zu Schritt a rückführt.

2. Verfahren nach Anspruch 1, **dadurch gekennzeichnet, daß** das Reagenz mit einer Carbonylgruppe ein Keton und vorzugsweise Aceton, Methylethylketon oder Methylisobutylketon ist.

3. Verfahren nach Anspruch 1 oder 2, **dadurch gekennzeichnet, daß** das Amid aus Acetamid, Proponiamid, n-Butyramid oder Isobutyramid gewählt ist.

4. Verfahren nach den Ansprüchen 1 bis 3, **dadurch gekennzeichnet, daß** man in Schritt a zusätzlich zu dem Amid ein Ammoniumsalz verwendet, das der Säure entspricht, von der sich das Amid ableitet.

## Claims

1. Process for the synthesis of azines from aqueous hydrogen peroxide from ammonia and from a reactant bearing a carbonyl group, **characterized in that**:
a) these reactants are placed in contact with a mixture containing an amide of a weak acid
b) the azine obtained is separated off
c) the mixture is treated by heating between 170 and 230°C to regenerate the initial quantity of the starting amide of stage a
d) the mixture obtained at the end of c is recycled to stage a.

2. Process according to Claim 1, **characterized in that** the reactant bearing a carbonyl group is a ketone and preferably acetone, methyl ethyl ketone or methyl isobutyl ketone.

3. Process according to Claim 1 or 2, **characterized in that** the amide is chosen from acetamide, proponiamide, n-butyramide and isobutyramide.

4. Process according to Claims 1 to 3, **characterized in that** in stage a, in addition to the amide, an ammonium salt corresponding to the acid from which the amide is derived is employed.
